(19) Europäisches Patentamt
European Patent Office
Office européen des brevets

(11)  **EP 2 977 075 B1**

(12)  **EUROPEAN PATENT SPECIFICATION**

(45) Date of publication and mention
of the grant of the patent:
**12.06.2019  Bulletin 2019/24**

(51) Int Cl.:
***A61M 27/00*** *(2006.01)*

(21) Application number: **14178203.7**

(22) Date of filing: **23.07.2014**

(54) **A Ureteral Stent**

Harnröhrenstent

Endroprothèse urétérale

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.01.2016  Bulletin 2016/04**

(73) Proprietor: **Coloplast A/S
3050 Humlebaek (DK)**

(72) Inventors:
• **Seguy, Sebastien
46300 Gourdon (FR)**
• **Beilvert, Thomas
24250 Saint Martial de Nabirat (FR)**
• **Millet, Jacques
24200 Sarlat la Canéda (FR)**
• **Bouguerara, Chaabane
2000 Frederiksberg C (DK)**
• **Chouarche, Olivier
24200 Vitrac (FR)**

(56) References cited:
**WO-A1-02/089893      US-A1- 2004 059 279**

## Description

### BACKGROUND

The present invention relates to a ureteral stent intended to maintain the flow of urine between the kidneys and the bladder of a patient.

In a person, urine secreted by the kidneys passes through the ureters to the bladder and is then evacuated from the body through the urethra during micturition. In a healthy person, the urine is evacuated from the kidney to the bladder in one direction, by means of peristaltic movements of the ureter.

Certain urological disorders or certain diseases may prevent this evacuation in the direction of the bladder. These disorders may in particular be due to the presence of a calculus or tumour or an obstruction of the pyeloureteral junction. In this case, the flow of urine to the bladder may be difficult or may no longer be possible at all. The urine remains in the kidney, which dilates and may cause nephritic colic. To remedy this disorder, a stent can be placed in the ureter in order to re-establish the function of the latter and to permit evacuation of the urine.

US 2004/0059279 discloses a ureteral stent according to the preamble of claim 1 including an elongated tubular segment extending toward the bladder from a kidney end region for placement in the renal cavity to a bladder end region.

Subject matter referred to as embodiments and/or inventions which are not claimed are not part of the invention.

### SUMMARY

The ureteral stent according to the invention as claimed in the appended claims includes a body and a tail, the body having a renal area for placement in a kidney of a patient, a ureteral area for placement in a ureter of the patient, and a proximal area arranged at a proximal end of the body of the stent, the tail having at least one thread configured to end in the bladder of said patient.

The present invention provides a stent which preserves the peristaltic movements of the ureter, thereby preventing reflux of urine in the direction of the kidneys. The stent is configured to avoid irritation of the bladder and irritation caused by friction in the ureter. The stent is provided to be better tolerated by patients. The tail has at least one thread permitting easy and optimal withdrawal of the stent. In one embodiment, the tail is additionally fixed to the stent to further optimize its functionality including guiding of urine without causing irritation while preventing reflux and permitting withdrawal of the stent.

The ureteral stent includes a proximal area that has a first flexibility greater than a second flexibility of the ureteral area of the stent. The tail is rigidly connected to the ureteral area, and extends beyond the proximal area.

The ureteral stent is useful for placement in a ureter of a patient suffering from a urological disorder or disease, such as a calculus, a tumour, or an obstruction of the pyeloureteral junction in particular. The stent extends as far as the kidney and has a curved renal area, the curved shape allowing the stent to be held in place in the kidney. The stent has a ureteral area configured to be inserted in the ureter of the patient. The ureteral area extends beyond the area in which the patient suffers from a disease or disorder, and it replaces the defective part of the ureter. One end of the stent is provided with a tail which comprises at least one thread configured to end in or near the bladder. The thread is suitable for permitting evacuation of the urine and has a sufficiently small diameter to be virtually physcially imperceptible to the patient.

The greater first flexibility of the proximal area over the second flexibility of the ureteral area greatly reduces or completely eliminates any discomfort in the patient caused by the presence of the stent. Moreover, the thread of the stent permits easier withdrawal of the stent for removal.

### BRIEF DESCRIPTION OF THE DRAWINGS

The present invention and its advantages will be better understood with reference to the detailed description and to the attached figures, in which:

- Figure 1 is a schematic view of one embodiment of a stent placed in a patient;

- Figures 2 and 3 are front views of examples of fixing a tail to a stent;

- Figure 4 is a profile view of part of one example of a ureteral stent;

- Figure 5 is a sectional view of part of the stent, according to one alternative example;

- Figure 6 is a cross-sectional view of the example of Figure 5;

- Figure 7 is a backside view of part of the stent, according to one example;

- Figure 8 is a cross-sectional view of the example of Figure 7;

- Figures 9 to 11 are sectional views of further examples;

- Figures 12, 14 and 16 to 18 are schematic perspective views of embodiments of the invention;

- Figure 13 is a cross-sectional view of the embodiment of Figure 12; and

- Figure 15 is a cross-sectional view of the embodiment of Figure 14.

## DETAILED DESCRIPTION

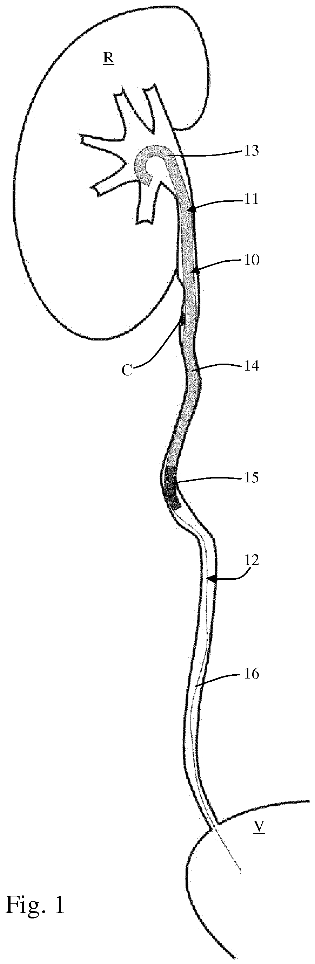

Figure 1 is a schematic view of one embodiment of a ureteral stent 10, placed in a patient presenting a calculus C. The ureteral stent 10 includes a body 11 and a tail 12. The body has a renal area 13 configured to be placed in a kidney R of a patient. The renal area 13 includes a curved end configured to prevent the stent from shifting in the ureter once in place. This part of the stent is physically imperceptible or virtually imperceptible to the patient and, as a result, does not cause any discomfort or pain.

The stent has a ureteral area 14 for placement in a ureter of the patient. Embodiments include stents manufactured with varying lengths of at least the ureteral area such as to accommodate variations in patient physiology. As has been indicated above, a ureteral stent of this kind is suitable for placement in a patient in cases of disease or other obstruction in the area of the ureter. The obstruction may be due to the presence of a calculus C, as is illustrated by Figure 1, a tumour or a constriction in particular. The length of the ureteral area 14 of the stent should be sufficient to ensure that, after placement of the stent, the body 11 thereof extends beyond the site of the calculus or the tumour in the direction of the bladder.

The body 11 of the stent additionally has a proximal area 15 which is rigidly connected to an end of the ureteral area 14, at the end away from the renal area 13. The proximal area 15 is situated in continuation of the ureteral area 14.

In one example, the tail 12 of the stent includes at least one thread or suture 16, which is configured to extend from the proximal area 15 in the direction of the bladder V, when the stent is placed in a patient.

The tail 12 is configured to end in the bladder and extends a few centimetres into the bladder when the stent is placed in a patient. In one embodiment, the tail extends 5 to 6 centimetres into the bladder.

In embodiments wherein the tail is formed by two or more threads, the threads may suitably be free and independent of one another, or alternatively fixed to one another, for example by means of a knot. The knot is suitably located near the proximal area 15, in which case it will be positioned in the ureter during the use of the stent. Alternatively, the knot is located near the end of the threads away from the proximal area, in which case it will be positioned in the bladder during the use of the stent. Other ways of connecting the threads to one another are also acceptable, such as, but not limited to braiding.

Providing a thread, even of small diameter, has several effects. One of the effects is to permit the flow of urine from the kidney R to the bladder V without permitting flow in the opposite direction from the bladder to the kidney. This reduces or eliminates problems concerning, in particular, the sense of having to pass urine.

Another effect of the thread is that it causes a dilation of the ureter, even if the thread has a small diameter in relation to the ureter. Such dilation permits easier and therefore less painful evacuation of the calculi.

In one embodiment, the ureteral area 14 and the proximal area 15 are tubular and cylindrical, that is to say they have a substantially annular cross section, with a longitudinal channel 17 defining a lateral wall 18. In one embodiment, the lateral wall has, at least in some areas, through-openings (not shown) which allow the urine to flow from outside the stent to the inside, and vice versa. In one embodiment, the longitudinal channel 17 opens out at the end of the proximal area 15, near the tail 12. In one embodiment, the renal area 13 also has a channel and openings passing through the wall. The channel serves, during the placement of the stent, for introduction of a guide and, during the use of the stent, for evacuation of urine.

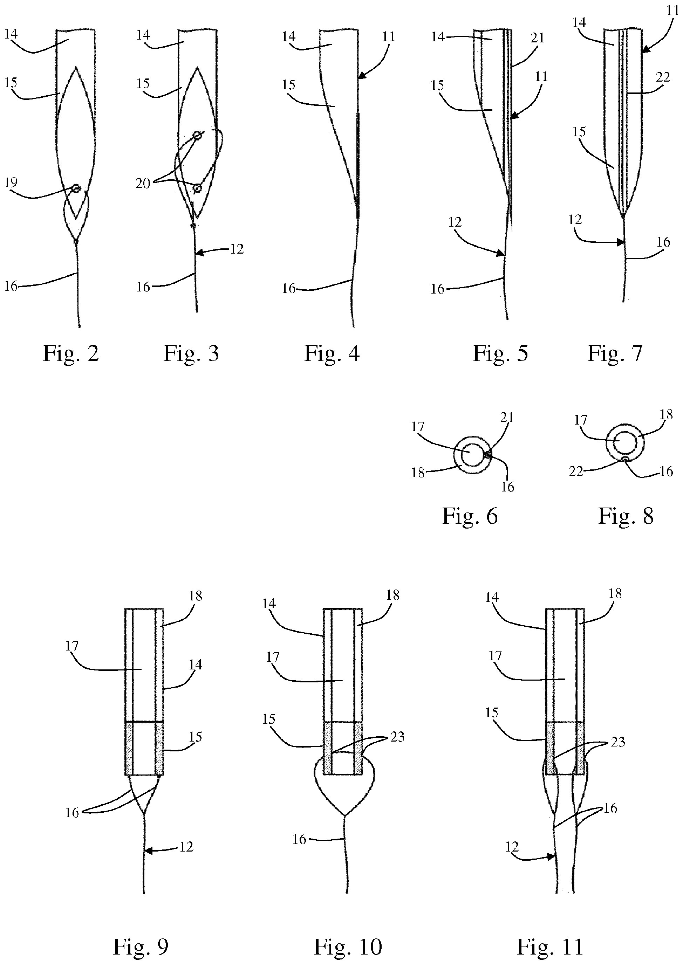

In examples illustrated in Figures 2 to 8, the proximal area 15 has a bevelled shape, this shape providing flexibility to the proximal area. In the embodiments illustrated in Figures 2 to 6, the tail 12 of the stent includes a thread 16 which is fixed in the proximal area 15 of the stent.

In this application, flexibility is defined here as the resistance to elastic deformation of a body. The more a body is flexible, the less the force that has to be applied to it to obtain a given deflection. In the present specification, stiffness or rigidity is considered the inverse of flexibility.

The flexibility can be measured in the following way: a specimen which flexibility is being determined is placed on two punctiform supports that are separated by a distance L. A downward force P is applied in the centre of the specimen. The deformation $U_y$ of the specimen is measured by measuring the movement of the centre of the specimen under the effect of the force P. This deformation, as a function of the force, allows a curve to be established. The coefficient of flexibility is defined as being the slope of the tangent to this curve, at the origin.

From the mathematical point of view, the coefficient of flexibility is defined by

$$K = \frac{L^3}{48} \cdot \frac{P}{|U_y|}$$

In one embodiment, the coefficient of flexibility of the proximal area of the stent is less than or equal to 200 N mm$^2$.

The more flexible the stent, the greater the flexibility and the lower the coefficient of flexibility.

Flexibility of the proximal area is suitably obtained by use of flexible materials and/or by providing the proximal area with a shape that gives it flexibility.

In one embodiment. the proximal area of the stent is configured to allow the body of the stent to match the shape of a ureter, in particular in the non-rectilinear parts of the ureter, during movements of the patient, especially movements caused by respiration. The stent and the ureter are configured to allow relative shifting between them without irritating or injuring the ureter. For this purpose, the proximal area of the stent is sufficiently flexible to be able to follow the ureter without inflicting injuries on the latter.

A suitable material for producing the proximal area can be chosen among different types of polymers such as polyurethane, copolymers such as the polyether block amide "PEBA", polyvinyl chloride, polyamides or silicone in particular, or more generally from among the materials mentioned below for the ureteral area.

In one embodiment, the ureteral area of the stent has a flexibility allowing it to adapt to the sinuosities of the ureter. Materials suitable for producing the stent include polymers such as polyurethane, copolymers such as polyether block amide known by the name PEBA, polyamides, silicone, polyolefins sold under the names Infuse, Vistamaxx™, Queo™ or Notio™; polyamides, PVC, thermoplastic polyurethanes, aromatic polyethers, aromatic and aliphatic polyesters having a Shore hardness of generally between 25 and 95; compounds based on thermoplastic elastomers, vulcanized thermoplastic elastomers, mixtures and alloys based on thermoplastic polyurethane, polymers and copolymers sold under the names Thermoflex™, Hytril™, Arnitel™, EVA, and thermoplastic elastomers known by their acronyms SIS, SEBS, SEPS, SEEPS, SBS, SIBS or SIBSTAR.

In embodiments, an external diameter of the ureteral stent is between 1.5 mm and 4 mm.

Suitable materials for the thread (or suture) include: polyethylene, polyamide, polyester, silk, steel, resorbable material (such as polyglactin acid), high-density polypropylene, meta-aramid and para-aramid, such as Kevlar™ or Nomex™.

In embodiments, the thread is configured to have a diameter ranging between 0.16 mm and 1.3 mm. In one embodiment, the diameter is substantially equal to 0.2 mm.

In one example illustrated in Figure 2, an end of the proximal area 15 of the stent has a through-opening 19. A thread 16 passes through the through-opening 19 and forms a loop, which is fixed to another part of the thread, for example by a knot.

In one example, the through-opening 19 is located relatively close to a free end of the proximal area 15, the free end defined as the end of the proximal area 15 away from the ureteral area 14.

In one example illustrated in Figure 3, the proximal area 15 has two openings 20 arranged at different distances from the free end of the proximal area 15. The thread 16 passes through the two openings 20, and one of the ends of the thread is fixed to another part of the thread. The fixation is suitably obtained by a knot, by adhesive bonding or by a combination of these.

One advantage of the example of Figure 3 is that it avoids rolling-up of the stent during withdrawal and unintentional tearing of the proximal area when the thread is pulled. It should be noted that the openings 20 are suitably placed with one in the proximal area 15 and the other in the ureteral area 14.

In one example illustrated in Figure 4, the thread 16 is arranged in a continuation of the tapered end of the proximal area 15. The thread is suitably provided separately from the body 11 of the stent and configured for subsequent attachment thereto. The tail is suitably fixed by adhesive bonding or by welding. In one embodiment, one or more threads are fixed by ultrasonic welding. In this context, it is possible to use an ultrasound-generating cone in the body of the stent and in this way weld the thread.

In one embodiment, the thread 16 is suitably produced at the same time as the body 11 of the stent, for example by co-extrusion or drawing of the material forming the body. Thereby, there is practically no transition between the body 11 of the stent and, in particular, between the proximal area 15 and the tail 12 of the stent.

In examples illustrated in Figures 5 to 8, a thread 16 opens out at the tapered end of the proximal area 15 with a gentle, or smooth, transition between the body 11 of the stent and the tail 12.

In the examples of Figures 5 and 6, the proximal area 15 includes a channel 21 in which the thread 16 is arranged. The thread is suitably held in the channel 21 by adhesive bonding or welding. In embodiments, the channel 21 suitably extends into the ureteral area 14.

In the examples illustrated in Figures 7 and 8, the proximal area 15 includes a groove 22 in which the thread 16 of the tail is arranged. The thread is suitably fixed to the body 11 by welding or adhesive bonding. In embodiments, the groove 22 suitably extends into the ureteral area 14.

In Figures 9 to 18, a first flexibility of the proximal area 15 is not provided by the bevelled shape but instead by the material or cut-outs made in this proximal area.

In the examples illustrated in Figures 9 to 11, the first flexibility of the proximal area 15 is provided by use of a flexible material. In the embodiment illustrated in Figure 9, the tail 12 has two strands of thread 16, which are fixed to a free end of the proximal area. The fixation is suitably obtained by adhesive bonding or welding. The two strands join to form one thread. In other embodiments, the threads do not join so that tail 12 includes more than one thread 16. In one embodiment, more than two threads are used and fixed to the proximal area 15.

In one example illustrated in Figure 10, the proximal area 15 has two through-openings 23 arranged substantially at the same distance from a free end of the proximal area. The thread 16 forming the tail passes through these through-openings 23 and forms a loop. The loop can be closed by a knot and/or by adhesive bonding. It is also possible to provide more than two through-openings.

In one example illustrated in Figure 11, the tail 12 includes two threads 16 and each thread 16 of passes through a single through-opening 23. It is possible to provide a tail formed by a single thread passing through a single opening or, by contrast, more than two threads.

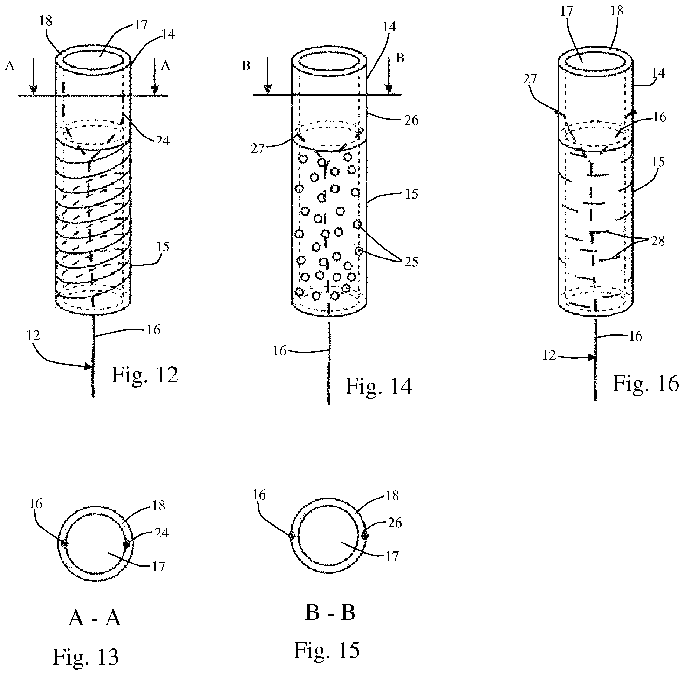

In embodiments illustrated in Figures 12 to 18, a first flexibility of the proximal area 15 is provided by removing material from the proximal area, as is explained in detail below.

In one embodiment illustrated in Figures 12 and 13, the proximal area 15 is formed by a spiral. This spiral shape provides the proximal area of the stent with high flexibility. In this embodiment, the tail 12 is not fixed to the proximal area 15, but to the ureteral area 14. At least part of the ureteral area 14 includes one or more internal grooves 24. A thread 16 of the tail is suitably arranged in each groove and is fixed therein. The fixation is suitably obtained by adhesive bonding or welding.

In the embodiment illustrated in Figures 14 and 15, the proximal area 15 includes a plurality of through-openings 25. At least one effect of the openings is that they make the proximal area more flexible. The ureteral area 14 has, at least in a part near the proximal area 15, two or more external longitudinal grooves 26 and two through-openings 27 connected to the external grooves 26. At least one thread 16 forming the tail passes through the openings 27 and is placed in the external grooves 26. The tail continues beyond the proximal area 15. The threads arranged in the external grooves 26 are fixed to the body of the stent by knots, adhesive bonding and/or welding. Other ways of fixing the threads to the body are also acceptable.

During normal use of the stent of this embodiment, urine is able to flow freely along the tail 12 without the tail interfering with the proximal area 15. During withdrawal of the stent, the pull applied to the tail acts on the ureteral area 14, which makes it possible to exert a certain amount of force without deforming the proximal area 15. Moreover, the proximal area retains its flexibility during withdrawal of the stent.

In one embodiment illustrated in Figure 16, the proximal area 15 includes transverse slits 28 provided to make the proximal area flexible. The ureteral area 14 includes at least two through-openings 27 arranged near the proximal area 15. A thread 16 forming the tail 12 passes through these openings 27 and is fixed to the body of the stent. The fixation is suitably obtained by a knot, welding and/or adhesive bonding.

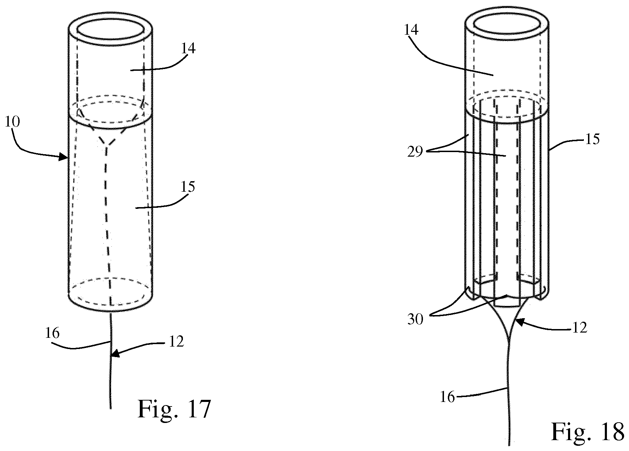

Figure 17 shows one embodiment of a stent 10 in which the proximal area 15 has a wall thickness that decreases towards a free end of the proximal area. The reduction in wall thickness increases the flexibility of the proximal area.

The tail 12 is fixed to the ureteral area 14. The fixation can be obtained by adhesive bonding, welding, coextrusion or by a knot.

In embodiments, the different shapes and different ways of fixing the tail 12 to the body 11 of the stent 10 may be combined. The external groove 26 may suitably be applied to any of the embodiments illustrated in Figures 12 to 17. The same applies to the other ways of fixing the tail to the body.

In one embodiment illustrated in Figure 18, the proximal area 15 is formed by tongues 29 each having a through-opening 30 placed near the free end of the proximal area 15. The tail 12 is provided by thread 16 which passes through the through-openings 30 of the tongues 29 and connects all of these. The tail 12 continues beyond the proximal area 15.

In this embodiment, the tongues 29 are flexible during normal use of the stent. Urine can flow along the tail 12 of the stent without the possibility of a reflux movement.

When the stent of this embodiment is withdrawn, a pull applied to the tail effectively draws the tongues 29 together. This prevents the tongues from rolling up and reduces the external diameter of the stent at the end of the body of the stent to make withdrawal of the stent easier.

A stent has been described which permits evacuation of urine from the patient while at the same time preventing reflux of the urine in the direction towards the kidneys. The stent also dilates the ureter, which permits easier evacuation of calculi. The stent is virtually imperceptible to the patient because the proximal area is flexible. The thread is fine (of small diameter) and flexible, which means that it is practically unnoticed by the patient. Moreover, the tail is fixedly secured to the body of the stent, which allows for easy withdrawal of the stent.

**Claims**

1. A ureteral stent (10) comprising:

   a body (11) having a renal area (13) configured to be placed in a kidney of a patient, a ureteral area (14) connected to the renal area (13) and configured to be placed in a ureter of the patient, and a proximal area (15) connected to the ureteral area (14) and located at a proximal end of the body (11); and

   a tail (12) comprising a thread (16) connected to the stent (10); wherein the proximal area (15) is provided with a first flexibility that is greater than a second flexibility of the ureteral area (14) of the stent and in that the tail (12) is fixed to the ureteral area (14) and is configured to extend from the proximal area (15) such that it extends beyond the proximal area in the direction of the bladder when the stent (10) is placed in a patient, **characterized in that** the ureteral area (14) includes a groove (24,26) into which the tail (12) is connected.

2. The ureteral stent according to claim 1, wherein the ureteral area (14) comprises one or more through-openings (27), and the tail (12) is fixed to the body (11) of the stent (10) via one or more of the through-openings (27).

3. The ureteral stent according to claim 1 or 2, wherein the tail (12) is fixed to the body (11) by a knot.

4. The ureteral stent according to claim 1 or 2, wherein the tail (12) is fixed to the body (11) by adhesive bonding.

5. The ureteral stent according to claim 1 or 2, wherein the tail (12) is fixed to the body (11) by welding.

6. The ureteral stent according to claim 2, wherein the tail (12) passes through two or more of the through-openings (27).

7. The ureteral stent according to claim 1, wherein the groove (26) is arranged outside the body (11) of the stent (10).

8. The ureteral stent according to claim 1, wherein the one or more grooves (24) are arranged inside the body (11) of the stent (10).

**Patentansprüche**

1. Urethraler Stent (10), umfassend:

einen Körper (11) mit einem renalen Bereich (13), der dazu ausgestaltet ist, in eine Niere eines Patienten platziert zu werden, einem urethralen Bereich (14), der mit dem renalen Bereich (13) verbunden und dazu ausgestaltet ist, in den Harnleiter des Patienten platziert zu werden, und einem proximalen Bereich (15), der mit dem urethralen Bereich (14) verbunden und an einem proximalen Ende des Körpers (11) angeordnet ist, und
einen Schwanz (12), der einen mit dem Stent (10) verbundenen Faden (16) umfasst, wobei der proximale Bereich (15) mit einer ersten Flexibilität versehen ist, die größer als eine zweite Flexibilität des urethralen Bereichs (14) des Stents ist, und dass der Schwanz (12) am urethralen Bereich (14) befestigt und dazu ausgestaltet ist, sich vom proximalen Bereich (15) zu erstrecken, so dass er sich über den proximalen Bereich in die Richtung der Blase erstreckt, wenn der Stent (10) in einem Patienten platziert ist,
**dadurch gekennzeichnet, dass** der urethrale Bereich (14) eine Nut (24, 26) aufweist, in die der Schwanz (12) verbunden ist.

2. Urethraler Stent nach Anspruch 1, wobei der urethrale Bereich (14) eine oder mehrere Durchgangsöffnungen (27) umfasst und der Schwanz (12) über eine oder mehrere der Durchgangsöffnungen (27) am Körper (11) des Stents (10) befestigt ist.

3. Urethraler Stent nach Anspruch 1 oder 2, wobei der Schwanz (12) durch einen Knoten am Körper (11) befestigt ist.

4. Urethraler Stent nach Anspruch 1 oder 2, wobei der Schwanz (12) durch eine Klebeverbindung am Körper (11) befestigt ist.

5. Urethraler Stent nach Anspruch 1 oder 2, wobei der Schwanz (12) durch Schweißen am Körper (11) befestigt ist.

6. Urethraler Stent nach Anspruch 2, wobei der Schwanz (12) durch zwei oder mehr der Durchgangsöffnungen (27) geht.

7. Urethraler Stent nach Anspruch 1, wobei die Nut (26) außerhalb des Körpers (11) des Stents (10) angeordnet ist.

8. Urethraler Stent nach Anspruch 1, wobei die eine oder die mehreren Nuten (24) innerhalb des Körpers (11) des Stents (10) angeordnet sind.

**Revendications**

1. Endoprothèse urétérale (10), comprenant :

un corps (11) ayant une zone rénale (13) configurée pour être placée dans le rein d'un patient, une zone urétérale (14) connectée à la zone rénale (13) et configurée pour être placée dans un uretère du patient, et une zone proximale (15) connectée à la zone urétérale (14) et située à une extrémité proximale du corps (11) ; et
une queue (12) comprenant un fil (16) connecté à l'endoprothèse (10) ; la zone proximale (15) étant dotée d'une première souplesse qui est supérieure à une deuxième souplesse de la zone urétérale (14) de l'endoprothèse et en ce que la queue (12) est fixée à la zone urétérale (14) et est configurée pour s'étendre depuis la zone proximale (15) de manière à s'étendre au-delà de la zone proximale dans la direction de la vessie lorsque l'endoprothèse (10) est placée dans un patient,
**caractérisée en ce que**
la zone urétérale (14) comporte une rainure (24, 26) dans laquelle est connectée la queue (12).

2. Endoprothèse urétérale selon la revendication 1, dans laquelle la zone urétérale (14) comprend une ou plusieurs ouvertures traversantes (27), et la queue (12) est fixée au corps (11) de l'endoprothèse (10) par le biais d'une ou plusieurs des ouvertures traversantes (27).

3. Endoprothèse urétérale selon la revendication 1 ou 2, dans laquelle la queue (12) est fixée au corps (11) par un noeud.

4. Endoprothèse urétérale selon la revendication 1 ou 2, dans laquelle la queue (12) est fixée au corps (11) par liaison adhésive.

5. Endoprothèse urétérale selon la revendication 1 ou 2, dans laquelle la queue (12) est fixée au corps (11) par soudage.

6. Endoprothèse urétérale selon la revendication 2, dans laquelle la queue (12) passe à travers deux ou plus de deux des ouvertures traversantes (27).

7. Endoprothèse urétérale selon la revendication 1, dans laquelle la rainure (26) est disposée à l'extérieur du corps (11) de l'endoprothèse (10).

8. Endoprothèse urétérale selon la revendication 1, dans laquelle la ou les rainures (24) sont disposées à l'intérieur du corps (11) de l'endoprothèse (10).

Fig. 1

Fig. 2  Fig. 3  Fig. 4  Fig. 5  Fig. 7

Fig. 6  Fig. 8

Fig. 9  Fig. 10  Fig. 11

Fig. 12

Fig. 14

Fig. 16

A - A

Fig. 13

B - B

Fig. 15

Fig. 17

Fig. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040059279 A